(19) Europäisches Patentamt
European Patent Office
Office européen des brevets

(11) Veröffentlichungsnummer: **0 114 224**
**A1**

(12) # EUROPÄISCHE PATENTANMELDUNG

(21) Anmeldenummer: 83111310.5

(22) Anmeldetag: 12.11.83

(51) Int. Cl.³: C 07 C 125/065
C 07 C 155/02, C 07 D 213/64
A 01 N 47/12

(30) Priorität: 17.12.82 CH 7370/82
21.09.83 CH 5131/83

(43) Veröffentlichungstag der Anmeldung:
01.08.84 Patentblatt 84/31

(84) Benannte Vertragsstaaten:
BE CH DE FR GB IT LI NL

(71) Anmelder: F. HOFFMANN-LA ROCHE & CO.
Aktiengesellschaft

CH-4002 Basel(CH)

(72) Erfinder: Zurflüh, René, Dr.
Dachslenbergstrasse 54
CH-8180 Bülach(CH)

(74) Vertreter: Aschert, Hubert et al,
Grenzacherstrasse 124 Postfach 3255
CH-4002 Basel(CH)

(54) **Benzoesäureester und Verfahren zu deren Herstellung.**

(57) Verbindungen der allgemeinen Formel

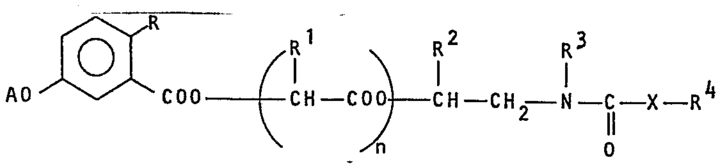

worin A eine Gruppe (a) oder (b)

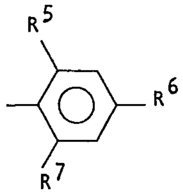
(a)

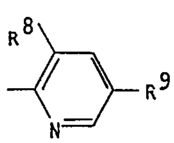
(b)

R Halogen, Nitro oder Cyano,
$R^1$ Wasserstoff oder Methyl,
$R^2$ Wasserstoff, Methyl oder Aethyl,
$R^3$ Wasserstoff oder Methyl,
$R^4$ -Alkyl oder 2-Chloräthyl,
$R^5$ Wasserstoff, Halogen oder Trifluormethyl,
$R^6$ Halogen oder Trifluormethyl,
$R^7$ Wasserstoff oder Halogen.
$R^8$ Wasserstoff oder Chlor,
$R^9$ Chlor oder Trifluormethyl,
n die Zahl 0 oder 1
und X Sauerstoff oder Schwefel bedeuten
Verfahren zu deren Herstellung, Unkrautbekämpfungsmittel, die diese Verbindungen als Wirkstoffe enthalten, sowie die Verwendung solcher Verbindungen bzw. Mittel zur Bekämpfung von Unkräutern.

EP 0 114 224 A1

Croydon Printing Company Ltd.

F.Hoffmann-La Roche & Co. Aktiengesellschaft,Basel,Schweiz

RAN 6102/39

# Benzoesäureester und Verfahren zu deren Herstellung

Die Erfindung betrifft Benzoesäureester der allgemeinen Formel

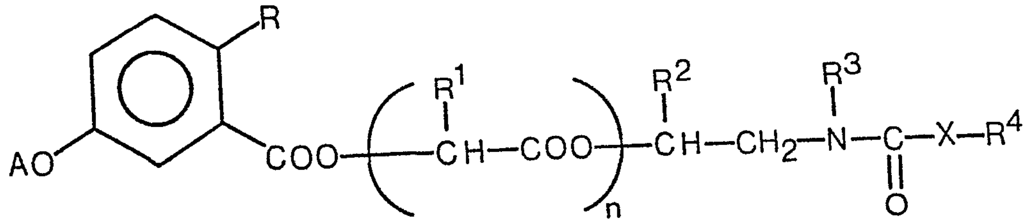

I

worin A eine Gruppe (a) oder (b)

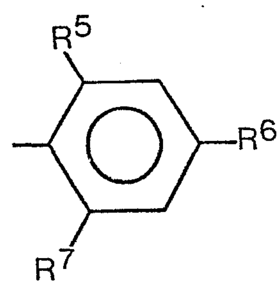

(a)

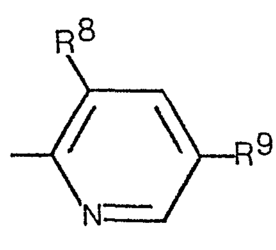

(b)

R Halogen, Nitro oder Cyano,
$R^1$ Wasserstoff oder Methyl,
$R^2$ Wasserstoff, Methyl oder Aethyl,
$R^3$ Wasserstoff oder Methyl,
$R^4$ $C_{1-4}$-Alkyl oder 2-Chloräthyl,
$R^5$ Wasserstoff, Halogen oder Trifluormethyl,
$R^6$ Halogen oder Trifluormethyl,
$R^7$ Wasserstoff oder Halogen,
$R^8$ Wasserstoff oder Chlor,
$R^9$ Chlor oder Trifluormethyl,
n die Zahl 0 oder 1
und X Sauerstoff oder Schwefel bedeuten.

Die Verbindungen der Formel I besitzen herbizide Eigenschaften und eignen sich somit als (Wirkstoffe von) Unkrautbekämpfungsmittel(n). Somit umfasst die Erfindung auch Unkrautbekämpfungsmittel, die mindestens eine Verbindung der Formel I als Wirkstoff enthalten, Verfahren zur Herstellung dieser Verbindungen sowie die Verwendung solcher Verbindungen bzw. Mittel zur Bekämpfung von Unkräutern.

In der obigen Formel I umfasst der Ausdruck "Halogen" Fluor, Chlor, Brom und Jod. Unter "$C_{1-4}$-Alkyl" sind sowohl geradkettige als auch verzweigte Alkylgruppen zu verstehen, wie Methyl, Aethyl, n-Propyl, Isopropyl, n-Butyl, Isobutyl und tert. Butyl.

Da asymmetrische Kohlenstoffatome im Molekül vorhanden sein können, können die Verbindungen der Formel I in optisch aktiver Form auftreten. Die Formel I soll demnach die möglichen optischen Isomeren sowie die Racemate umfassen.

Unabhängig voneinander bedeuten A vorzugsweise eine Gruppe (a), R vorzugsweise Chlor oder Nitro, $R^2$ vorzugsweise Wasserstoff, $R^3$ vorzugsweise Wasserstoff, $R^4$ vorzugsweise Methyl oder Aethyl, $R^5$ vorzugsweise Chlor, $R^6$ vorzugsweise

Chlor oder Trifluormethyl, $R^7$ vorzugsweise Wasserstoff oder Chlor, und X vorzugsweise Sauerstoff.

Besonders bevorzugte Verbindungen der Formel I sind:

2-[5-(o-Chlor-α,α,α-trifluor-p-tolyloxy)-2-nitro-benzoyloxy]äthylcarbaminsäure-methylester,

2-/2-[5-(o-Chlor-α,α,α-trifluor-p-tolyloxy)-2-nitro-benzoyloxy]-propionyloxy/propylcarbaminsäure-methylester,

2-/[5-(o-Chlor-α,α,α-trifluor-p-tolyloxy)-2-nitro-benzoyloxy]acetyloxy/äthylcarbaminsäure-methylester,

2-/2-[5-(o-Chlor-α,α,α-trifluor-p-tolyloxy)-2-nitro-benzoyloxy]-propionyloxy/äthylcarbaminsäure-methylester und

2-/2-[5-(o-Chlor-α,α,α-trifluor-p-tolyloxy)-2-chlor-benzoyloxy]-propionyloxy/ propylcarbaminsäure-methylester.

Weitere Vertreter der Verbindungen der Formel I sind:

2-[5-(3-Chlor-5-trifluormethyl-2-pyridyloxy)-2-nitro-benzoyloxy]äthylcarbaminsäure-äthylester,

2-[5-(3,5-Dichlor-2-pyridyloxy)-2-nitro-benzoyloxy]-äthylcarbaminsäure-äthylester,

2-[5-(5-Trifluormethyl-2-pyridyloxy)-2-nitro-benzoyloxy]-äthylcarbaminsäure-äthylester,

2-/[5-(o-Chlor-α,α,α-trifluor-p-tolyloxy)-2-nitro-benzoyloxy]acetyloxy/äthylcarbaminsäure-äthylester,

2-[5-(o-Chlor-α,α,α-trifluor-p-tolyloxy)-2-chlor-benzoyloxy]äthylcarbaminsäure-äthylester

und 2-[5-(o-Chlor-α,α,α-trifluor-p-tolyloxy)-2-cyano-benzoyloxy]äthylcarbaminsäure-äthylester.

Das erfindungsgemässe Verfahren zur Herstellung der Verbindungen der Formel I ist dadurch gekennzeichnet, dass man

a) eine substituierte Benzoesäure der allgemeinen Formel

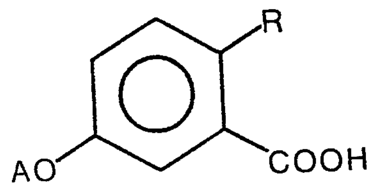

II

worin A und R die oben angegebenen Bedeutungen besitzen,

oder ein Salz bzw. reaktionsfähiges Derivat davon, mit einer Verbindung der allgemeinen Formel

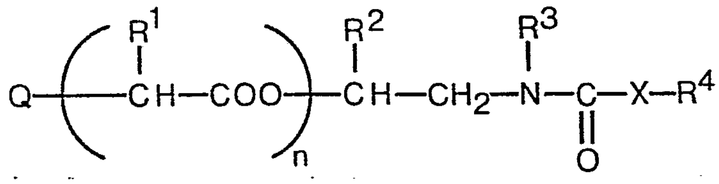

III

worin $R^1$, $R^2$, $R^3$, $R^4$, n und X die oben angegebenen Bedeutungen besitzen und Q eine Abgangsgruppe bedeutet,

umsetzt,

b) zwecks Herstellung der Verbindungen der Formel I, worin R Nitro bedeutet, ein substituiertes Benzoesäurederivat der allgemeinen Formel

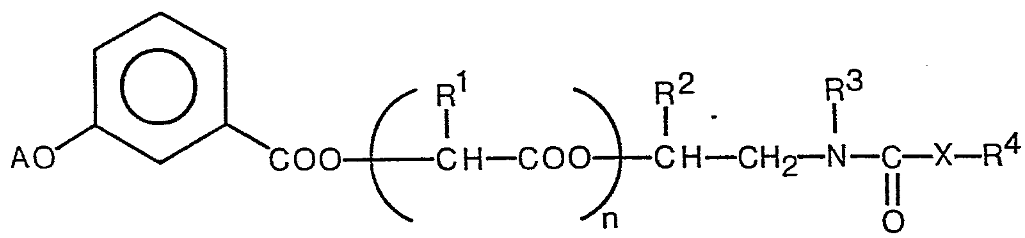

IV

worin A, $R^1$, $R^2$, $R^3$, $R^4$, n und X die oben angegebenen Bedeutungen besitzen,

nitriert,

c) zwecks Herstellung der Verbindungen der Formel I, worin A eine Gruppe (a) und R Nitro oder Cyano bedeuten, ein substituiertes Phenol der allgemeinen Formel

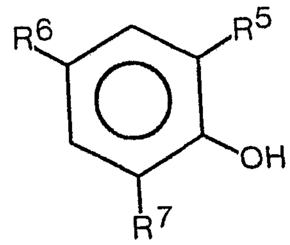

V

worin $R^5$, $R^6$ und $R^7$ die oben angegebenen Bedeutungen besitzen,

oder ein Alkalimetallsalz davon, mit einem substituierten Halogenbenzoesäurederivat der allgemeinen Formel

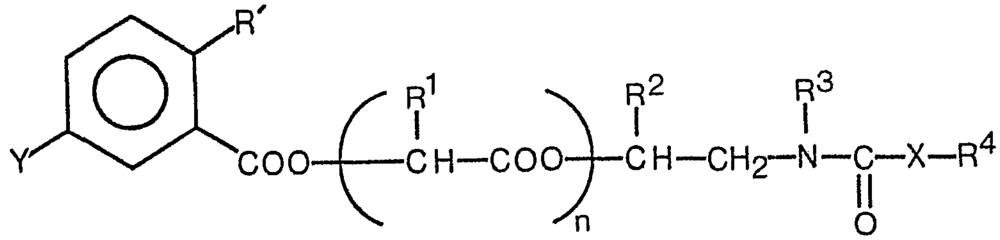

VI

worin R' Nitro oder Cyano bedeutet, $R^1$, $R^2$, $R^3$, $R^4$, n und X die oben angegebenen Bedeutungen besitzen und Y Halogen, insbesondere Fluor, Chlor oder Brom, bedeutet,

umsetzt, oder

d) zwecks Herstellung der Verbindungen der Formel I, worin A eine Gruppe (b) bedeutet, ein substituiertes Hydroxybenzoesäurederivat der allgemeinen Formel

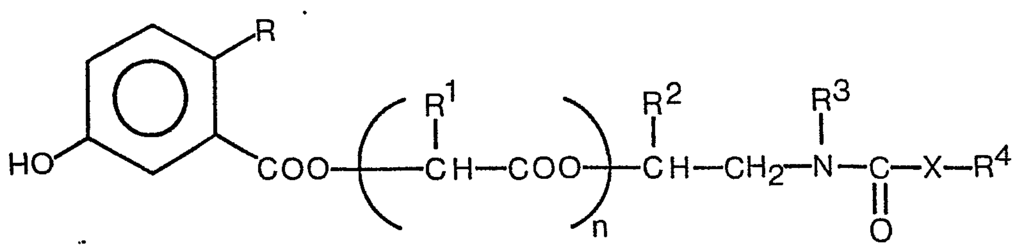

VII

worin R, $R^1$, $R^2$, $R^3$, $R^4$, n und X die oben angegebenen Bedeutungen besitzen,

mit einem substituierten Halogenpyridin der allgemeinen Formel

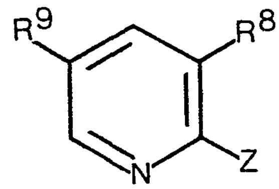

VIII

worin $R^8$ und $R^9$ die oben angegebenen Bedeutungen besitzen und Z Halogen, insbesondere Fluor oder Chlor, bedeutet,

umsetzt.

Bei der Verfahrensvariante a) handelt es sich um eine Veresterung, die nach an sich bekannten Methoden durchgeführt werden kann, z.B. gemäss den nachfolgenden Varianten $a_1$), $a_2$) und $a_3$):

| | Edukt | Reagens |
|---|---|---|
| $a_1$) | Freie Säure der Formel II, insbesondere aber Salze davon | Verbindung der Formel III, worin Q beispielsweise für Chlor, Brom, Jod, Mesyloxy oder Tosyloxy steht |
| $a_2$) | Halogenid, Imidazolid oder Anhydrid der Säure der Formel II | Verbindung der Formel III, worin Q für Hydroxy steht |
| $a_3$) | Freie Säure der Formel II | Verbindung der Formel III, worin Q für Hydroxy steht. |

Bei der Verfahrensvariante $a_1$) bedeutet der Ausdruck "Salz" beispielsweise ein Alkalimetallsalz, z.B. das Natrium-, Kalium- oder Lithiumsalz, ein Erdalkalimetallsalz, z.B. das Magnesium-, Calcium- oder Bariumsalz, ein Salz einer organischen Base, z.B. ein Mono-, Di- oder Trialkylammoniumsalz oder ein Pyridiniumsalz, oder das Ammoniumsalz. Der Ausdruck "Abgangsgruppe" für Q steht vorzugsweise für Chlor, Brom, Jod, Mesyloxy oder Tosyloxy.

Die Veresterung der Säure der Formel II, vorzugsweise in Form eines Salzes, mit der Verbindung der Formel III wird vorzugsweise in einem inerten Lösungsmittel und bei Temperaturen von etwa -20°C bis 100°C durchgeführt. Ein besonders bevorzugter Temperaturbereich ist derjenige zwischen 0°C und 40°C. Es kommen als Lösungsmittel vorzugsweise inerte organische Lösungsmittel in Frage. Hierzu gehören vorzugsweise Aether, z.B. Diäthyläther und Tetrahydrofuran, chlorierte Kohlenwasserstoffe, z.B. Dichlormethan und Chloroform, und Dimethylformamid.

Bei Verwendung der freien Säure der Formel II wird die Umsetzung zweckmässigerweise in Gegenwart einer Base bzw. eines Säureacceptors durchgeführt. Man kann dazu alle üblicherweise verwendbaren anorganischen und organischen säurebindenden Mittel benützen, zu denen vorzugsweise Alkalimetall- und Erdalkalimetallcarbonate- und -bicarbonate, und tertiäre Amine, z.B. Triäthylamin, Dimethylanilin und Pyridin, gehören.

Die Umsetzung nach Verfahrensvariante $a_2$) wird zweckmässigerweise in einem inerten Lösungsmittel, wie einem Aether, z.B. Diäthyläther, einem Kohlenwasserstoff, z.B. n-Hexan, oder einem chlorierten Kohlenwasserstoff, z.B. Dichlormethan oder Chloroform, und bei Raumtemperatur oder erhöhter Temperatur, z.B. bis zur Rückflusstemperatur des Reaktionsgemisches, durchgeführt. Ein bevorzugter Temperaturbereich ist derjenige zwischen 0°C und 40°C, insbesondere zwischen 10°C und 20°C.

Bei Verwendung eines Säurehalogenides der Säure der Formel II wird die Umsetzung zweckmässigerweise in Gegenwart eines säurebindenden Mittels durchgeführt. Als geeignet erweisen sich anorganische Basen, z.B. Alkalimetall- und Erdalkalimetallcarbonate und -bicarbonate, sowie organische Basen, z.B. tertiäre Amine, insbesondere Triäthylamin oder Pyridin. Das Säurehalogenid ist vorzugsweise das Säurechlorid.

Bei der Verfahrensvariante $a_3$) wird die Umsetzung zweckmässigerweise in einem inerten organischen Lösungsmittel, wie einem Aether, z.B. Tetrahydrofuran, oder einem chlorierten Kohlenwasserstoff, z.B. Dichlormethan oder Chloroform, und bei Temperaturen zwischen 0°C und 70°C durchgeführt. Ein bevorzugter Temperaturbereich ist derjenige zwischen 10°C und 30°C. Die Umsetzung erfolgt zweckmässigerweise in Gegenwart eines sauren Katalysators bzw. eines kondensierenden Mittels, wie beispielsweise Schwefelsäure, Salzsäure, p-Toluolsulfonsäure, Dicyclohexylcarbodiimid oder Carbonyldiimidazol.

Die Nitrierung gemäss Verfahrensvariante b) erfolgt zweckmässigerweise mittels Salpetersäure bzw. Salpetersäure enthaltender Gemische oder Lösungen, oder mittels Kaliumnitrat. Beispiele solcher Gemische bzw. Lösungen sind Gemische von Salpetersäure und Schwefelsäure, Lösungen von Salpetersäure in Eisessig und Lösungen von konzentrierter Salpetersäure in chlorierten Kohlenwasserstoffen, z.B. Aethylenchlorid. Bei der Verwendung von Kaliumnitrat wird dieses vorzugsweise als Lösung in konzentrierter Schwefelsäure eingesetzt. Die Nitrierung wird zweckmässigerweise bei Temperaturen zwischen 0°C und der Raumtemperatur durchgeführt.

Die Verfahrensvariante c) oder d) kann zweckmässigerweise durchgeführt werden, indem man das Phenol der Formel V bzw. VII in ein Alkalimetallsalz, beispielsweise unter Verwendung von Natriumhydrid oder Kaliumhydroxid mit azeotroper Wasserentfernung, überführt und anschliessend das Salz mit dem Halogenid der Formel VI bzw. VIII reagieren lässt. Die letztgenannte Reaktionsstufe wird zweckmässigerweise in einem dipolaren aprotischen Lösungsmittel, z.B. Dimethylformamid oder Dimethylsulfoxid, oder in Pyridin, bei Temperaturen zwischen 50°C und 150°C, vorzugsweise zwischen 70°C und 110°C, vorgenommen.

Die Isolierung und Reinigung der so hergestellten Verbindungen der Formel I können nach an sich bekannten Methoden erfolgen.

Für den Fall, dass asymmetrische Kohlenstoffatome im Molekül der Formel I vorhanden sind und keine gezielte Synthese zur Isolierung reiner optischer Isomerer durchgeführt wird, fällt das Produkt normalerweise als Racemat an. Die Isomeren können nach an sich bekannten Methoden aufgetrennt werden. Gewünschtenfalls können allerdings die optischen Isomeren durch Synthese aus entsprechenden optisch aktiven Ausgangsmaterialien hergestellt werden.

Die Ausgangsmaterialien der Formeln II, V und VIII sowie Salze bzw. reaktionsfähige Derivate der Säuren der Formel II und Alkalimetallsalze der Phenole der Formel V sind entweder bekannt oder können in an sich bekannter Weise hergestellt werden.

Die Ausgangsmaterialien der Formel III können in an sich bekannter Weise, z.B. nach folgendem Reaktionsschema, hergestellt werden. Im Reaktionsschema besitzen die Symbole $R^1$, $R^2$, $R^3$, $R^4$, X und Q die oben angegebenen Bedeutungen und bedeutet Hal ein Halogenatom, insbesondere Chlor.

Reaktionsschema

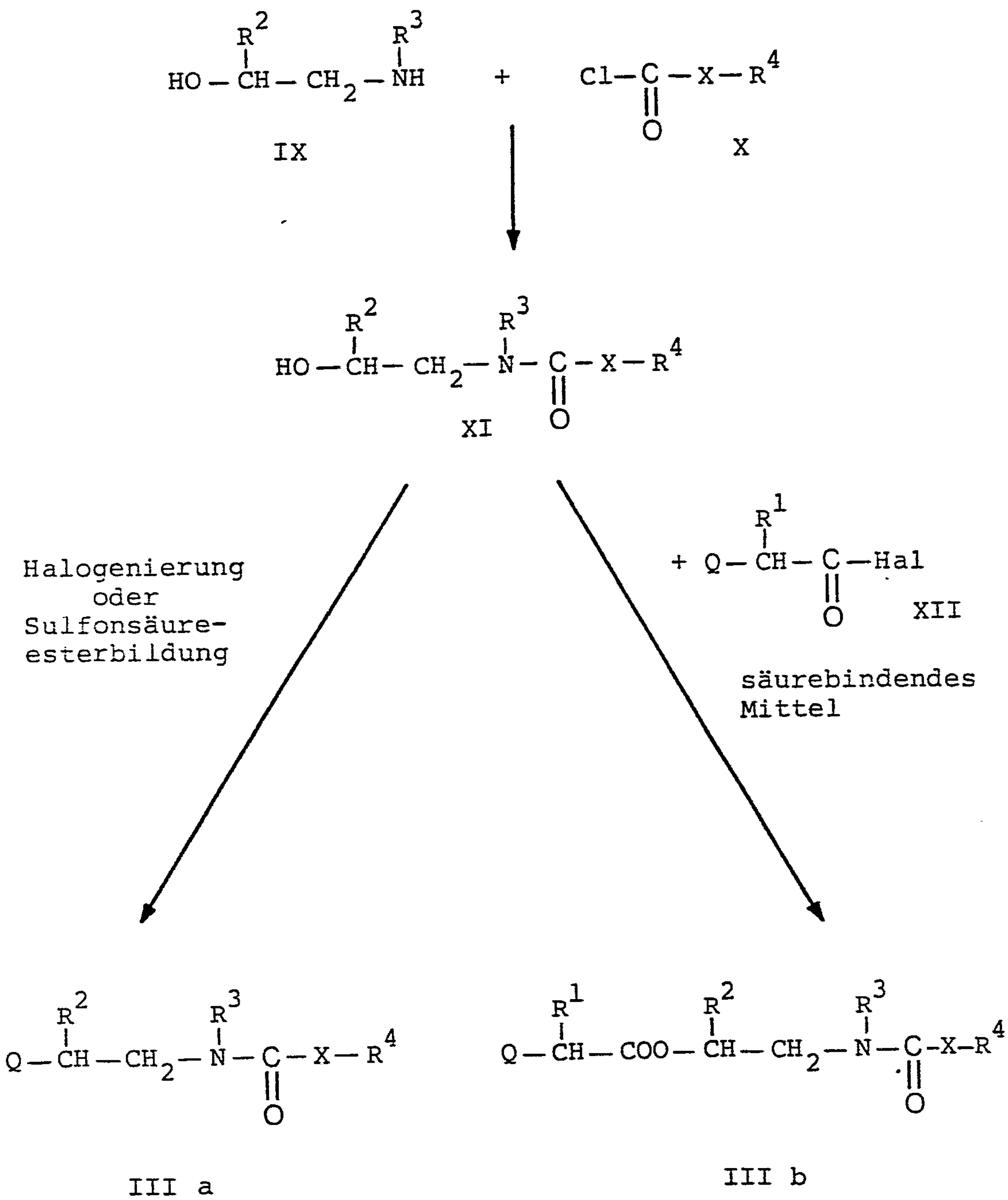


IX

X

XI

XII

III a

III b

Die Ausgangsmaterialien der Formeln IV, VI und VII sind neu. Sie können aus den entsprechenden Benzoesäuren der allgemeinen Formeln

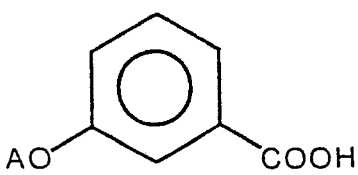 bzw. 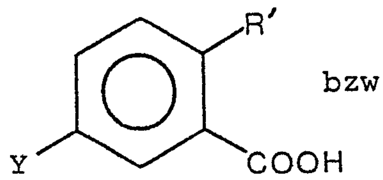 bzw. 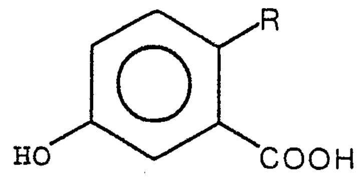

worin A, R', Y und R die oben angegebenen Bedeutungen besitzen,

oder Salzen bzw. reaktionsfähigen Derivaten davon, und einer Verbindung der Formel III, wie oben definiert, unter den bezüglich der obigen Verfahrensvariante a) angegebenen Reaktionsbedingungen hergestellt werden.

Die Verbindungen der Formel I besitzen herbizide Eigenschaften und eignen sich zur Bekämpfung von Unkräutern, insbesondere von Galium aparine, Amaranthus retroflexus, Datura stramonium, Ipomea spp, Abutilon theophrasti und Chenopodium album in Getreide, insbesondere Gersten-, Hafer- und Weizenkulturen sowie in Reis- und Sojakulturen. Besonders bevorzugt eignen sich die erfindungsgemässen Verbindungen zur Bekämpfung von Unkräutern in Reis-, Weizen- und Sojakulturen.

Im allgemeinen genügt eine Konzentration von 0,1 - 6 kg Wirkstoff der Formel I/ha, vorzugsweise 0,6 - 2 kg Wirkstoff der Formel I/ha, um den gewünschten herbiziden Effekt zu erzielen.

Die Verbindungen der Formel I sind sowohl Vorauflauf-Herbizide als auch Nachauflauf-Herbizide.

Das erfindungsgemässe Unkrautbekämpfungsmittel ist dadurch gekennzeichnet, dass es eine wirksame Menge mindestens einer Verbindung der Formel I, wie oben definiert,

sowie Formulierungshilfsstoffe enthält. Das Mittel enthält zweckmässigerweise zumindest einen der folgenden Formulierungshilfsstoffe: feste Trägerstoffe; Lösungs- bzw. Dispersionsmittel; Tenside (Netz- und Emulgiermittel); Dispergatoren (ohne Tensidwirkung); und Stabilisatoren. Unter Verwendung solcher und anderer Hilfsstoffe können die Verbindungen der Formel I, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I sind im allgemeinen wasserunlöslich und können nach den für wasserunlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln und/oder von Dispergatoren, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte ali-

phatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungsmittel vorzugsweise Flammpunkte von mindestens 30°C und Siedepunkt von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzol-sulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kathionische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäuren, Natriumsalze von Maleinsäurenanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel z.B. Epichlorhydrin, Phenylglycidäther und Soyaepoxide; Antioxidantien z.B. Gallussäureester und Butylhydroxytoluol; UV-Absorber, z.B. substituierte Benzophenone, Diphenylacrylonitrilsäureester und Zimtsäureester; und Deaktivatoren, z.B. Salze der Aethylendiaminotetraessigsäure und Polyglykole.

Die erfindungsgemässen Unkrautbekämpfungsmittel können zusätzlich zu den Verbindungen der Formel I Synergisten und andere Wirkstoffe, z.B. Insektizide, Akarizide, Bakterizide, anderweitige Herbizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,1 und 99 Gewichtsprozent, vorzugsweise zwischen 5 und 75 Gewichtsprozent einer bzw. mehrerer Verbindungen der Formel I als Wirkstoff(e). Sie

können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 10 und 75 Gewichtsprozent, insbesondere zwischen 25 und 50 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,1 bis 10 Gewichtsprozent, insbesondere ca. 1 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine Verbindung der Formel I, mit festem Trägerstoff vermischt werden, z.B. durch Zusammenmahlen; oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindung der Formel I kann auch mit einem Tensid und einem festen Trägerstoff zur Bildung eines netzbaren Pulvers vermischt werden, welches in Wasser dispergierbar ist, oder sie kann mit einem festen vorgranulierten Trägerstoff zur Bildung eines granulatförmigen Produktes vermischt werden.

Wenn gewünscht, kann die Verbindung der Formel I in

einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung bzw. einem erfindungsgemässen Unkrautbekämpfungsmittel behandelt.

Die nachfolgenden Beispiele dienen zur näheren Erläuterung der Erfindung.

I. Herstellung der Wirkstoffe der Formel I:

Beispiel 1

30,0 g 5-(o-Chlor-α,α,α-trifluor-p-tolyloxy)-2-nitro-benzoesäure werden in 41,3 g Thionylchlorid gelöst, und die Lösung wird während 2,5 Stunden auf Rückflusstemperatur erhitzt. Nach Abdestillieren des überschüssigen Thionylchlorids und Trocknen am Hochvakuum erhält man 31,8 g 5-(o-Chlor-α,α,α-trifluor-p-tolyloxy)-2-nitro-benzoesäurechlorid. Davon werden 5,3 g in 40 ml Methylenchlorid gelöst und tropfenweise zu einer eiskalten Lösung von 1,85 g 2-Hydroxypropylcarbaminsäure-methylester in 20 ml Methylenchlorid und 1,3 g Pyridin gegeben. Dann lässt man während 16 Stunden bei Raumtemperatur nachreagieren. Zur Aufarbeitung wird die Reaktionslösung unter vermindertem Druck eingeengt und der Rückstand in Diäthyläther aufgenommen. Die Aetherphase wird nacheinander mit 2N Natronlauge, Wasser, halbgesättigter Kochsalzlösung und gesättigter Kochsalzlösung gewaschen, über wasserfreiem Natriumsulfat getrocknet und unter vermindertem Druck eingedampft.

Der ölige Rückstand wird an Kieselgel mit n-Hexan/Diäthyläther (1:1) chromatographisch gereinigt. Man erhält den 2-[5-(o-Chlor-α,α,α-trifluor-p-tolyloxy)-2-nitro-benzoyloxy]propylcarbaminsäure-methylester; $n_D^{20}$ 1,5280.

In analoger Weise erhält man ausgehend von

5-(o-Chlor-α,α,α-trifluor-p-tolyloxy)-2-nitro-benzoesäurechlorid und N-(2-Hydroxyäthyl)-N-methylcarbaminsäure-methylester den N-[2-[5-(o-Chlor-α,α,α-trifluor-p-tolyloxy)-2-nitro-benzoyloxy]-äthyl]-N-methyl-carbaminsäure-methylester; $n_D^{20}$ 1,5420.

5-(o-Chlor-α,α,α-trifluor-p-tolyloxy)-2-nitro-benzoesäurechlorid und 2-Hydroxyäthylcarbaminsäure-methylester den 2-[5-(o-Chlor-α,α,α-trifluor-p-tolyloxy)-2-nitro-

benzoyloxy]äthylcarbaminsäure-methylester; $n_D^{20}$ 1,547.

5-(o-Chlor-α,α,α-trifluor-p-tolyloxy)-2-nitro-benzoesäurechlorid und N-(2-Hydroxyäthyl)-N-methyl-thiolcarbaminsäure-äthylester den N-/2-[5-(o-Chlor-α,α,α-trifluor-p-tolyloxy)-2-nitro-benzoyloxy]-äthyl/-N-methyl-thiolcarbaminsäure-äthylester; $n_D^{20}$ 1,5582.

5-(o-Chlor-α,α,α-trifluor-p-tolyloxy)-2-nitro-benzoesäurechlorid und 2-Hydroxyäthylcarbaminsäure-äthylester den 2-[5-(o-Chlor-α,α,α-trifluor-p-tolyloxy)-2-nitro-benzoyloxy]äthylcarbaminsäure-äthylester; Smp. 88,5-89,5°C.

5-(o-Chlor-α,α,α-trifluor-p-tolyloxy)-2-chlor-benzoesäurechlorid und 2-Hydroxyäthylcarbaminsäure-methylester den 2-[5-(o-Chlor-α,α,α-trifluor-p-tolyloxy)-2-chlor-benzoyloxy]äthylcarbaminsäure-methylester; Smp. 70-72°C.

5-(o-Chlor-α,α,α-trifluor-p-tolyloxy)-2-jod-benzoesäurechlorid und 2-Hydroxyäthylcarbaminsäure-methylester den 2-[5-(o-Chlor-α,α,α-trifluor-p-tolyloxy)-2-jod-benzoyloxy]äthylcarbaminsäure-methylester; Smp. 73-75°C.

5-(5-Trifluormethyl-2-pyridyloxy)-2-nitro-benzoesäurechlorid und 2-Hydroxyäthylcarbaminsäure-methylester den 2-[5-(5-Trifluormethyl-2-pyridyloxy)-2-nitro-benzoyloxy]äthylcarbaminsäure-methylester; Smp. 77-80°C.

5-(3,5-Dichlor-2-pyridyloxy)-2-nitro-benzoesäurechlorid und 2-Hydroxyäthylcarbaminsäure-methylester den 2-[5-(3,5-Dichlor-2-pyridyloxy)-2-nitro-benzoyloxy]äthylcarbaminsäure-methylester; $n_D^{23}$1,5745.

5-(o-Chlor-α,α,α-trifluor-p-tolyloxy)-2-cyano-benzoesäurechlorid und 2-Hydroxyäthylcarbaminsäure-methylester den 2-[5-(o-Chlor-α,α,α-trifluor-p-tolyloxy)-2-cyano-benzoyloxy]äthylcarbaminsäure-methylester;

5-(o-Chlor-α,α,α-trifluor-p-tolyloxy)-2-nitro-benzoesäurechlorid und 2-Hydroxyäthylcarbaminsäure-2-chloräthylester den 2-[5-(o-Chlor-α,α,α-trifluor-p-tolyloxy)-2-nitro-benzoyloxy]äthylcarbaminsäure-2-chloräthylester;

5-(3-Chlor-5-trifluormethyl-2-pyridyloxy)-2-nitrobenzoesäurechlorid und 2-Hydroxyäthylcarbaminsäure-methylester den 2-[5-(3-Chlor-5-trifluormethyl-2-pyridyloxy)-2-nitro-benzoyloxy]äthylcarbaminsäure-methylester; Smp. 78-80°C.

Beispiel 2

0,8 g Natriumhydrid (55% in Oel) werden durch zweimaliges Waschen mit n-Hexan vom Oel befreit und dann mit 20 ml Dimethylformamid überdeckt. Dazu tropft man innert 45 Minuten eine Lösung von 6,7 g 5-(o-Chlor-α,α,α-trifluor-p-tolyloxy)-2-nitro-benzoesäure in 30 ml Dimethylformamid, wobei die Temperatur auf ca. 30°C ansteigt. Nun lässt man 6 Stunden bei Raumtemperatur nachreagieren und tropft anschliessend innert 15 Minuten eine Lösung von 5,0 g 2-(2-Brompropionyloxy)propylcarbaminsäure-methylester in 30 ml Dimethylformamid zu. Unter Rühren lässt man 24 Stunden bei 50°C nachreagieren. Das Reaktionsgemisch wird auf Eiswasser gegossen und mit Diäthyläther extrahiert. Die Aetherphase wird der Reihe nach mit 10% Natronlauge, Wasser und gesättigter Kochsalzlösung gewaschen, über wasserfreiem Natriumsulfat getrocknet und unter vermindertem Druck eingedampft. Der Rückstand wird an Kieselgel mit n-Hexan/Diäthyläther (1:1) chromatographisch gereinigt. Man erhält den 2-/2-[5-(o-Chlor-α,α,α-trifluor-p-tolyloxy)-2-nitro-benzoyloxy]-propionyloxy/propylcarbaminsäure-methylester; $n_D^{20}$ 1,5720.

In analoger Weise erhält man ausgehend von

5-(o-Chlor-α,α,α-trifluor-p-tolyloxy)-2-nitro-benzoesäure und N-[2-(2-Brompropionyloxy)-äthyl]-N-methyl-carbaminsäure-methylester den N-/2-{2-[5-(o-Chlor-α,α,α-trifluor-p-tolyloxy)-2-nitro-benzoyloxy]-propionyloxy}-äthyl_7-N-methyl-carbaminsäure-methylester; $n_D^{20}$ 1,5210.

5-(o-Chlor-α,α,α-trifluor-p-tolyloxy)-2-nitro-benzoesäure und 2-(Bromacetyloxy)äthylcarbaminsäure-methylester den 2-/[5-(o-Chlor-α,α,α-trifluor-p-tolyloxy)-2-nitro-benzoyloxy]acetyloxy/äthylcarbaminsäure-methylester; $n_D^{20}$ 1,5425.

5-(o-Chlor-α,α,α-trifluor-p-tolyloxy)-2-nitro-benzoesäure und 2-(2-Brompropionyloxy)äthylcarbaminsäure-methylester den 2-/2-[5-(o-Chlor-α,α,α-trifluor-p-tolyloxy)-2-nitro-benzoyloxy]-propionyloxy/äthylcarbaminsäure-methylester; $n_D^{20}$ 1,5359.

5-(o-Chlor-α,α,α-trifluor-p-tolyloxy)-2-chlorbenzoesäure und 2-(2-Brompropionyloxy)propylcarbaminsäure-methylester den 2-/2-[5-(o-Chlor-α,α,α-trifluor-p-tolyloxy)-2-chlor-benzoyloxy]-propionyloxy/propylcarbaminsäure-methylester; $n_D^{20}$ 1,5205.

5-(o-Chlor-α,α,α-trifluor-p-tolyloxy)-2-fluor-benzoesäure und 2-(2-Brompropionyloxy)propylcarbaminsäure-methylester den 2-/2-[5-(o-Chlor-α,α,α-trifluor-p-tolyloxy)-2-fluor-benzoyloxy]-propionyloxy/propylcarbaminsäure-methylester.

5-(o,o-Dichlor-α,α,α-trifluor-p-tolyloxy)-2-nitro-benzoesäure und 2-(2-Brompropionyloxy)propylcarbaminsäure-methylester den 2-/2-[5-(o,o-Dichlor-α,α,α-trifluor-p-tolyloxy)-2-nitro-benzoyloxy]-propionyloxy/propylcarbaminsäure-methylester.

5-(o,o,p-Trichlorphenoxy)-2-nitro-benzoesäure und 2-(2-Brompropionyloxy)propylcarbaminsäure-methylester den

2-/2-[5-(o,o,p-Trichlorphenoxy)-2-nitro-benzoyloxy]-propionyloxy/ propylcarbaminsäure-methylester.

5-(o,p-Dichlorphenoxy)-2-nitro-benzoesäure und 2-(2-Brompropionyloxy)propylcarbaminsäure-methylester den 2-/2-[5-(o,p-Dichlorphenoxy)-2-nitro-benzoyloxy]-propionyloxy/propylcarbaminsäure-methylester.

Beispiel 3

4,17 g 2-[5-(o-Chlor-α,α,α-trifluor-p-tolyloxy)-benzoyloxy]äthylcarbaminsäure-methylester und 10 ml konzentrierte Schwefelsäure werden bei Eistemperatur vorgelegt. Alsdann wird eine Lösung von 1,01 g Kaliumnitrat in 10 ml konzentrierter Schwefelsäure unter Rühren bei 0-5°C während 30 Minuten zugetropft. Nach erfolgter Zugabe wird das Eisbad entfernt, und nach 15 Minuten wird das Reaktionsgemisch auf ca. 100 g Eis gegossen und mit Toluol extrahiert. Die Extrakte werden mit Wasser und gesättigter Kochsalzlösung gewaschen, über wasserfreiem Natriumsulfat getrocknet und eingedampft. Nach chromatographischer Reinigung erhält man den 2-[5-(o-Chlor-α,α,α-trifluor-p-tolyloxy)-2-nitro-benzoyloxy]äthylcarbaminsäure-methylester; Smp. 73-75°C.

Beispiel 4

0,2 g Natriumhydrid (ca. 55% Dispersion in Oel) werden in 10 ml N,N-Dimethylformamid vorgelegt. Man tropft zum Gemisch bei Raumtemperatur 0,9 g 2-Chlor-4-trifluormethylphenol in 10 ml N,N-Dimethylformamid und lässt es während 16 Stunden bei 50°C rühren. Nun werden bei 90-100°C 1,4 g 2-(5-Fluor-2-nitro-benzoyloxy)äthylcarbaminsäure-äthylester gelöst in 10 ml N,N-Dimethylformamid, 10 mg Kupfer(I)-chlorid und 5 Tropfen Pyridin zugegeben. Dann lässt man das Reaktionsgemisch 4,5 Stunden bei ca. 100°C rühren.

Das Reaktionsgemisch wird auf Eiswasser gegossen und mit Diäthyläther extrahiert. Die Aetherphase wird mit 2N Natronlauge, Wasser und gesättigter Kochsalzlösung gewaschen, über wasserfreiem Natriumsulfat getrocknet und unter vermindertem Druck eingedampft. Der ölige Rückstand wird durch Chromatographie an Kieselgel mit n-Hexan/Aethylacetat gereinigt. Man erhält den 2-[5-(o-Chlor-α,α,α-trifluor-p-tolyloxy)-2-nitro-benzoyloxy]-äthylcarbaminsäure-äthylester; Smp. 84-87°C.

II. <u>Herstellung der Ausgangsmaterialien der Formeln III, IV und VI:</u>

## Beispiel 5

1,33 g 2-Hydroxypropylcarbaminsäure-methylester und 0,79 g Pyridin werden in 20 ml Diäthyläther gelöst und auf 0°C gekühlt. Nun werden 2,16 g 2-Brompropionsäurebromid innerhalb von 20 Minuten zugetropft, und das Gemisch wird noch 30 Minuten bei Eistemperatur gerührt. Zur Aufarbeitung wird auf Wasser gegossen und mit Diäthyläther extrahiert. Die Aetherphase wird mit Kochsalzlösung zweimal gewaschen, über wasserfreiem Natriumsulfat getrocknet und unter vermindertem Druck eingedampft. Man erhält den 2-(2-Brompropionyloxy)propylcarbaminsäure-methylester als farbloses Oel, das entweder ohne weitere Reinigung zur Reaktion eingesetzt werden kann oder durch Chromatographie an Kieselgel mit n-Hexan/Diäthyläther (1:1) weiter gereinigt werden kann; $n_D^{20}$ 1,4764.

In analoger Weise erhält man ausgehend von

N-(2-Hydroxyäthyl)-N-methyl-carbaminsäure-methylester und 2-Brompropionsäurebromid den N-[2-(2-Brompropionyloxy)-äthyl]-N-methyl-carbaminsäure-methylester; $^1$H-NMR ($CDCl_3$; 60 MHz) 4,35 ppm (q, 1H), 4,32 ppm (t, 2H), 3,75 ppm (s, 3H), 3,58 ppm (t, 2H), 3,0 ppm (s, 3H), 1,82 ppm (d, 3H).

BAD ORIGINAL

2-Hydroxyäthyl-carbaminsäure-methylester und 2-Bromacetylbromid den 2-(Bromacetyloxy)äthylcarbaminsäure-methylester; $n_D^{20}$ 1,4891.

2-Hydroxyäthyl-carbaminsäure-methylester und 2-Brompropionsäurebromid den 2-(2-Brompropionyloxy)äthylcarbaminsäure-methylester; $n_D^{20}$ 1,4799.

Beispiel 6

7,1 g 2-Hydroxyäthylcarbaminsäure-methylester und 5,1 g Pyridin werden in 100 ml Methylenchlorid vorgelegt. Zum Gemisch werden bei Raumtemperatur 18 g 3-(o-Chlor-α,α,α-trifluor-p-tolyloxy)-benzoylchlorid (hergestellt aus der entsprechenden Säure und Thionylchlorid) getropft. Man lässt das Reaktionsgemisch während 16 Stunden rühren, worauf unter vermindertem Druck eingeengt wird. Der Rückstand wird in Diäthyläther aufgenommen und die Aetherlösung mit 2N Salzsäure und Wasser gewaschen, über wasserfreiem Natriumsulfat getrocknet und eingedampft. Der Rückstand wird in Aethylacetat gelöst und das Produkt durch Zugabe von n-Hexan in der Kälte ausgefällt. Man erhält den 2-[5-(o-Chlor-α,α,α-trifluor-p-tolyloxy)-benzoyloxy]äthylcarbaminsäure-methylester; Smp. 80-81°C.

Beispiel 7

1,6 g 2-Hydroxyäthylcarbaminsäure-äthylester und 1,2 g Pyridin werden in 15 ml Methylenchlorid vorgelegt. Zum Gemisch werden bei Raumtemperatur 2,4 g 5-Fluor-2-nitrobenzoylchlorid (hergestellt aus 5-Fluor-2-nitrobenzoesäure und Thionylchlorid) getropft. Man lässt das Reaktionsgemisch während 16 Stunden bei Rückflusstemperatur reagieren, dann wird unter vermindertem Druck eingeengt. Der Rückstand wird in Diäthyläther aufgenommen und die Aetherlösung mit Wasser, 2N Natronlauge, halb-gesättigter- und gesättigter Kochsalzlösung gewaschen, getrocknet und unter vermindertem Druck eingeengt. Durch Chromatographie

an Kieselgel mit n-Hexan/Diäthyläther (1:1) erhält man den 2-(5-Fluor-2-nitro-benzoyloxy)äthylcarbaminsäure-äthylester als Feststoff.

III. Formulierungsbeispiele:

Beispiel 8

Zur Herstellung eines emulgierbaren Konzentrates werden die nachstehend aufgeführten Bestandteile miteinander vermischt:

| | |
|---|---|
| Wirkstoff der Formel I, z.B. 2-[5-(o-Chlor-α,α,α-trifluor-p-tolyloxy)-2-nitro-benzoyloxy]äthylcarbaminsäure-methylester | 250 g/l |
| N-Methyl-2-pyrrolidon | 300 g/l |
| Emulgator A[1)] | 100 g/l |
| Emulgator B[2)] | 25 g/l |
| Lösungsmittelgemisch aus Alkylbenzolen | auf 1000 ml |

1) Emulgator A: Emulgator bestehend aus 60 Teilen eines Blockpolymerisates aus Aethylenoxid und Propylenoxid, 20 Teilen des Calciumsalzes einer verzweigtkettigen Dodecylbenzolsulfonsäure und 20 Teilen eines Lösungsmittelgemisches aus Isobutanol und $C_{10}$-Alkylbenzolen.

2) Emulgator B: Gemisch aus 70 Teilen des Calciumsalzes einer verzweigtkettigen Dodecylbenzolsulfonsäure und 30 Teilen eines Lösungsmittelgemisches aus Isobutanol und $C_{10}$-Alkylbenzolen.

Das so erhaltene Konzentrat emulgiert spontan in Wasser. Die sich ergebende Emulsion eignet sich als gebrauchsfertige Spritzbrühe.

Beispiel 9

Zur Herstellung eines Spritzpulvers werden die nachstehend aufgeführten Bestandteile miteinander vermischt und hierauf in einer geeigneten Mühle feingemahlen:

| | Gewichtsteile |
|---|---|
| Wirkstoff der Formel I, mit einem Schmelzpunkt > 50°C | 50 |
| Hydratisierte Kieselsäure | 5 |
| Natrium-laurylsulfat | 1 |
| Natrium-lignosulfonat | 2 |
| Kaolin | 42 |

Das so erhaltene Pulver kann mit Wasser benetzt werden. Die entstandene Suspension eignet sich als gebrauchsfertige Spritzbrühe.

## Patentansprüche

1. Verbindungen der allgemeinen Formel

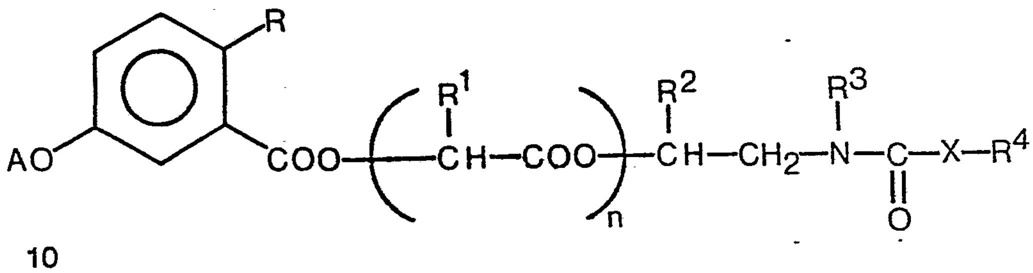

I

worin A eine Gruppe (a) oder (b)

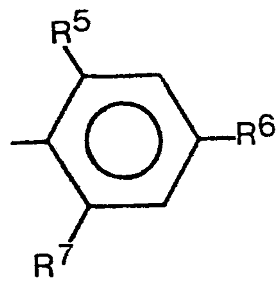

(a)

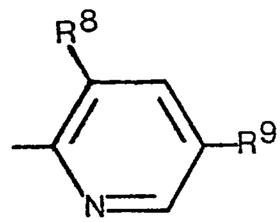

(b)

R Halogen, Nitro oder Cyano,
$R^1$ Wasserstoff oder Methyl,
$R^2$ Wasserstoff, Methyl oder Aethyl,
$R^3$ Wasserstoff oder Methyl,
$R^4$ $C_{1-4}$-Alkyl oder 2-Chloräthyl,
$R^5$ Wasserstoff, Halogen oder Trifluormethyl,
$R^6$ Halogen oder Trifluormethyl,
$R^7$ Wasserstoff oder Halogen,
$R^8$ Wasserstoff oder Chlor,
$R^9$ Chlor oder Trifluormethyl,
n die Zahl 0 oder 1

und X Sauerstoff oder Schwefel bedeuten.

2. Verbindungen nach Anspruch 1, worin A eine Gruppe (a), $R^5$ Chlor, $R^6$ Chlor oder Trifluormethyl und $R^7$ Wasserstoff oder Chlor bedeuten.

3. Verbindungen nach Anspruch 1 oder 2, worin R Nitro bedeutet.

4. Verbindungen nach einem der Ansprüche 1 bis 3, worin $R^2$ Wasserstoff bedeutet.

5. Verbindungen nach einem der Ansprüche 1 bis 4, worin $R^3$ Wasserstoff bedeutet.

6. Verbindungen nach einem der Ansprüche 1 bis 5, worin $R^4$ Methyl oder Aethyl bedeutet.

7. Verbindungen nach einem der Ansprüche 1 bis 6, worin X Sauerstoff bedeutet.

8. 2-[5-(o-Chlor-α,α,α-trifluor-p-tolyloxy)-2-nitro-benzoyloxy]äthylcarbaminsäure-methylester.

9. 2-/2-[5-(o-Chlor-α,α,α-trifluor-p-tolyloxy)-2-nitro-benzoyloxy]-propionyloxy/propylcarbaminsäure-methylester.

10. 2-/[5-(o-Chlor-α,α,α-trifluor-p-tolyloxy)-2-nitro-benzoyloxy]acetyloxy/äthylcarbaminsäure-methylester.

11. 2-/2-[5-(o-Chlor-α,α,α-trifluor-p-tolyloxy)-2-nitro-benzoyloxy]-propionyloxy/äthylcarbaminsäure-methylester.

12. 2-/2-[5-(o-Chlor-α,α,α-trifluor-p-tolyloxy)-2-chlor-benzoyloxy]-propionyloxy/propylcarbaminsäure-methylester.

13. Eine Verbindung nach Anspruch 1, ausgewählt aus:

2-[5-(o-Chlor-α,α,α-trifluor-p-tolyloxy)-2-nitro-benzoyloxy]propylcarbaminsäure-methylester,
N-/2-[5-(o-Chlor-α,α,α-trifluor-p-tolyloxy)-2-nitro-benzoyloxy]äthyl/-N-methyl-carbaminsäure-methylester,
N-/2-[5-(o-Chlor-α,α,α-trifluor-p-tolyloxy)-2-nitro-benzoyloxy]äthyl/-N-methyl-thiolcarbaminsäure-äthylester,
2-[5-(o-Chlor-α,α,α-trifluor-p-tolyloxy)-2-nitro-benzoyloxy]äthylcarbaminsäure-äthylester,
2-[5-(o-Chlor-α,α,α-trifluor-p-tolyloxy)-2-chlor-benzoyloxy]äthylcarbaminsäure-methylester,
2-[5-(o-Chlor-α,α,α-trifluor-p-tolyloxy)-2-jod-benzoyloxy]äthylcarbaminsäure-methylester,
2-[5-(5-Trifluormethyl-2-pyridyloxy)-2-nitro-benzoyloxy]äthylcarbaminsäure-methylester,
2-[5-(3,5-Dichlor-2-pyridyloxy)-2-nitro-benzoyloxy]äthylcarbaminsäure-methylester,
2-[5-(o-Chlor-α,α,α-trifluor-p-tolyloxy)-2-cyano-benzoyloxy]äthylcarbaminsäure-methylester,
2-[5-(3-Chlor-5-trifluormethyl-2-pyridyloxy)-2-nitro-benzoyloxy]äthylcarbaminsäure-methylester und
N-/2-{2-[5-(o-Chlor-α,α,α-trifluor-p-tolyloxy)-2-nitro-benzoyloxy]-propionyloxy}-äthyl/-N-methyl-carbaminsäure-methylester.

14. Eine Verbindung nach Anspruch 1, ausgewählt aus:

2-[5-(o-Chlor-α,α,α-trifluor-p-tolyloxy)-2-nitro-benzoyloxy]äthylcarbaminsäure-2-chloräthylester,
2-/2-[5-(o-Chlor-α,α,α-trifluor-p-tolyloxy)-2-fluor-benzoyloxy]-propionyloxy/propylcarbaminsäure-methylester,
2-/2-[5-(o,o-Dichlor-α,α,α-trifluor-p-tolyloxy)-2-nitro-benzoyloxy]-propionyloxy/propylcarbaminsäure-methylester,

2-/2-[5-(o,o,p-Trichlorphenoxy)-2-nitro-benzoyloxy]-propionyloxy/propylcarbaminsäure-methylester und

2-/2-[5-(o,p-Dichlorphenoxy)-2-nitro-benzoyloxy]-propionyloxy/propylcarbaminsäure-methylester.

15. Eine Verbindung nach Anspruch 1 als Unkrautbekämpfungsmittel.

16. Unkrautbekämpfungsmittel, dadurch gekennzeichnet, dass es eine wirksame Menge mindestens einer Verbindung der allgemeinen Formel

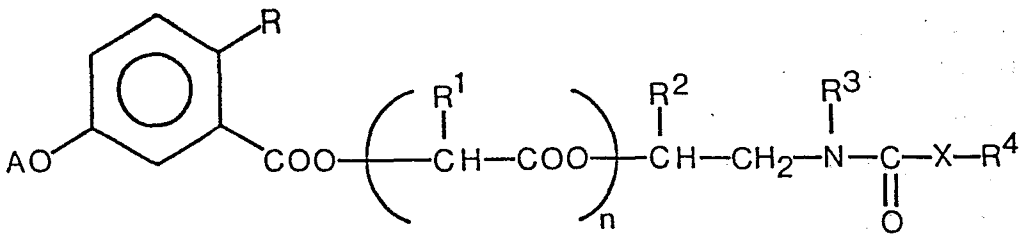

I

worin A eine Gruppe (a) oder (b)

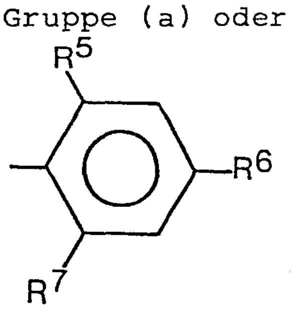

(a)

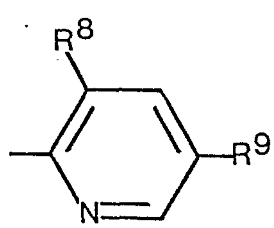

(b)

R Halogen, Nitro oder Cyano,

$R^1$ Wasserstoff oder Methyl,

$R^2$ Wasserstoff, Methyl oder Aethyl,

$R^3$ Wasserstoff oder Methyl,

$R^4$ $C_{1-4}$-Alkyl oder 2-Chloräthyl,

$R^5$ Wasserstoff, Halogen oder Trifluormethyl,

| | |
|---|---|
| $R^6$ | Halogen oder Trifluormethyl, |
| $R^7$ | Wasserstoff oder Halogen, |
| $R^8$ | Wasserstoff oder Chlor, |
| $R^9$ | Chlor oder Trifluormethyl, |
| n | die Zahl 0 oder 1 |
| und X | Sauerstoff oder Schwefel bedeuten, |

sowie Formulierungshilfsstoffe enthält.

17. Unkrautbekämpfungsmittel nach Anspruch 16, dadurch gekennzeichnet, dass es eine wirksame Menge 2-[5-(o-Chlor-α,α,α-trifluor-p-tolyloxy)-2-nitro-benzoyl-oxy]äthylcarbaminsäure-methylester oder 2-/2-[5-(o-Chlor-α,α,α-trifluor-p-tolyloxy)-2-nitro-benzoyloxy]-propionyloxy/propylcarbaminsäure-methylester oder 2-/[5-(o-Chlor-α,α,α-trifluor-p-tolyloxy)-2-nitro-benzoyloxy]-acetyloxy/äthylcarbaminsäure-methylester oder 2-/2-[5-(o-Chlor-α,α,α-trifluor-p-tolyloxy)-2-nitro-benzoyloxy]-propionyloxy/äthylcarbaminsäure-methylester oder 2-/2-[5-(o-Chlor-α,α,α-trifluor-p-tolyloxy)-2-chlor-benzoyloxy]-propionyloxy/propylcarbaminsäure-methylester sowie Formulierungshilfsstoffe enthält.

18. Verfahren zur Herstellung von Verbindungen der allgemeinen Formel

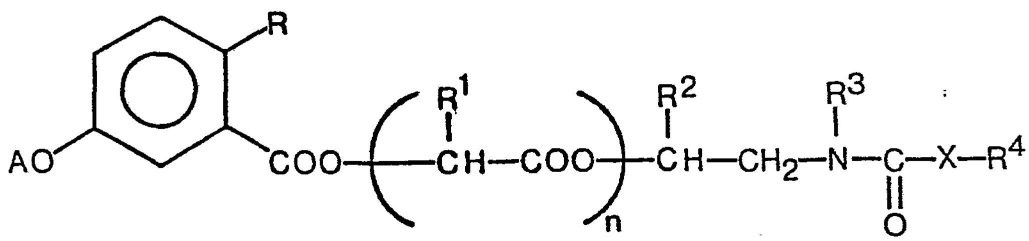

I

worin A eine Gruppe (a) oder (b)

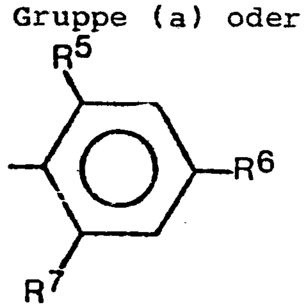

(a)

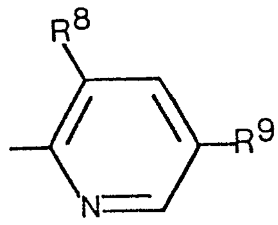 (b)

R Halogen, Nitro oder Cyano,

$R^1$ Wasserstoff oder Methyl,

$R^2$ Wasserstoff, Methyl oder Aethyl,

$R^3$ Wasserstoff oder Methyl,

$R^4$ $C_{1-4}$-Alkyl oder 2-Chloräthyl,

$R^5$ Wasserstoff, Halogen oder Trifluormethyl,

$R^6$ Halogen oder Trifluormethyl,

$R^7$ Wasserstoff oder Halogen,

$R^8$ Wasserstoff oder Chlor,

$R^9$ Chlor oder Trifluormethyl,

n die Zahl 0 oder 1

und X Sauerstoff oder Schwefel bedeuten,

dadurch gekennzeichnet, dass man

a) eine substituierte Benzoesäure der allgemeinen Formel

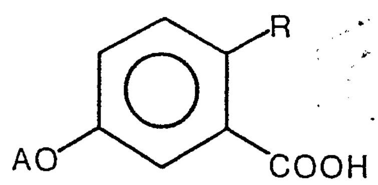 II

worin A und R die oben angegebenen Bedeutungen besitzen,

oder ein Salz bzw. reaktionsfähiges Derivat davon, mit einer Verbindung der allgemeinen Formel

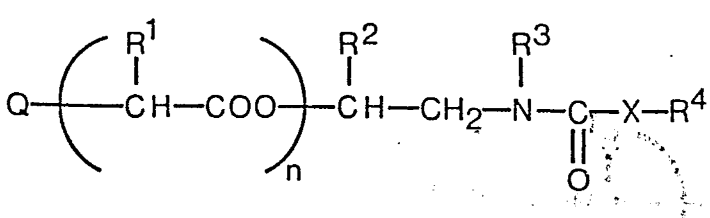 III

worin $R^1$, $R^2$, $R^3$, $R^4$, n und X die oben angegebenen Bedeutungen besitzen und Q eine Abgangsgruppe bedeutet,

umsetzt,

b) zwecks Herstellung der Verbindungen der Formel I, worin R Nitro bedeutet, ein substituiertes Benzoesäurederivat der allgemeinen Formel

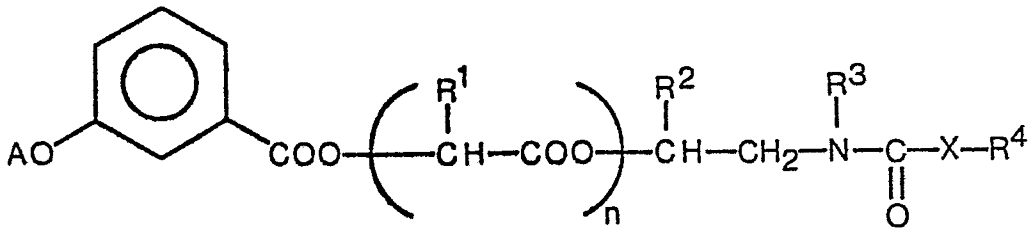

IV

worin A, $R^1$, $R^2$, $R^3$, $R^4$, n und X die oben angegebenen Bedeutungen besitzen,

nitriert,

c) zwecks Herstellung der Verbindungen der Formel I, worin A eine Gruppe (a) und R Nitro oder Cyano bedeuten, ein substituiertes Phenol der allgemeinen Formel

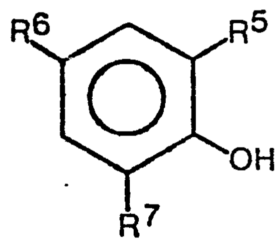

V

worin $R^5$, $R^6$ und $R^7$ die oben angegebenen Bedeutungen besitzen,

oder ein Alkalimetallsalz davon, mit einem substituierten Halogenbenzoesäurederivat der allgemeinen Formel

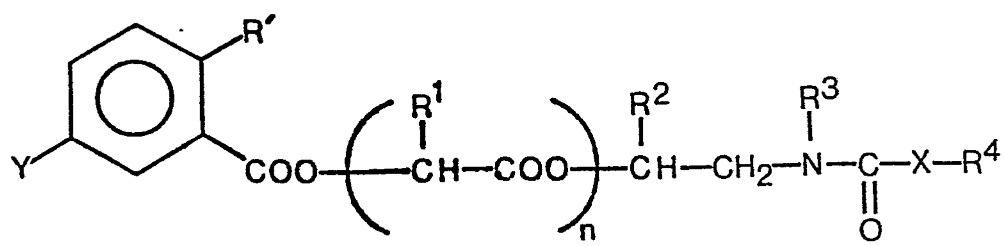

VI

worin R' Nitro oder Cyano bedeutet, $R^1$, $R^2$, $R^3$, $R^4$, n und X die oben angegebenen Bedeutungen besitzen und Y Halogen bedeutet,

umsetzt, oder

d) zwecks Herstellung der Verbindungen der Formel I, worin A eine Gruppe (b) bedeutet, ein substituiertes Hydroxybenzoesäurederivat der allgemeinen Formel

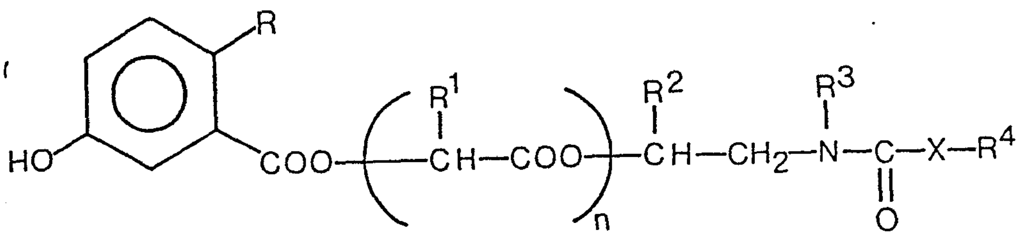

VII

worin R, $R^1$, $R^2$, $R^3$, $R^4$, n und X die oben angegebenen Bedeutungen besitzen,

mit einem substituierten Halogenpyridin der allgemeinen Formel

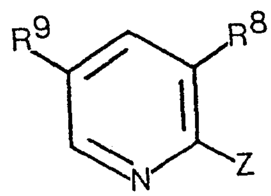

VIII

worin $R^8$ und $R^9$ die oben angegebenen Bedeutungen besitzen und Z Halogen bedeutet,

umsetzt.

19. Verfahren zur Bekämpfung von Unkräutern, dadurch gekennzeichnet, dass man das gegen Unkräuter zu schützende Gut und/oder die Unkräuter mit einer wirksamen Menge einer der in den Ansprüchen 1 bis 14 genannten Verbindungen bzw. eines in Anspruch 16 oder 17 genannten Mittels behandelt.

20. Verwendung einer der in den Ansprüchen 1 bis 14 genannten Verbindungen bzw. eines in Anspruch 16 oder 17 genannten Mittels zur Bekämpfung von Unkräutern.

***

Europäisches Patentamt

# EUROPÄISCHER RECHERCHENBERICHT

0114224

Nummer der Anmeldung

EP 83 11 1310

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | EP-A-0 066 106 (GAF CORP.)<br>* Ansprüche 6,7 *<br>----- | 1-20 | C 07 C 125/065<br>C 07 C 155/02<br>C 07 D 213/64<br>A 01 N 47/12 |
| | | | **RECHERCHIERTE SACHGEBIETE (Int. Cl. 3)** |
| | | | C 07 C 125/00 |

Der vorliegende Recherchenbericht wurde für alle Patentansprüche erstellt.

| Recherchenort | Abschlußdatum der Recherche | Prüfer |
|---|---|---|
| DEN HAAG | 27-03-1984 | GAUTIER R.H.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument